# EUROPEAN PATENT APPLICATION

(11) **EP 1 739 072 A1**
(43) Date of publication of application: **03.01.2007**
(21) Application number: 05012919.6
(22) Date of filing: 15.06.2005
(51) Int. Cl.: C07C 51/41, C07C 59/84

(54) **Polymorphic forms of dexketoprofen trometamol, preparation and pharmaceutical compositions thereof**

(71) Applicant: LABORATORIOS MENARINI S.A., 08912 Badalona, Barcelona (ES)
(72) Inventor: Bosch, Margarita, 08912 Badalona Barcelona (ES); Mannucci, Serena, 56100 san Piero a Grado(PISA) (IT); Torras, Ester, 08912 Badalona Barcelona (ES); Falorni, Roberto, 56100 San Piero a Grado (PISA) (IT); Gonzales, Josep Ma., 08912 Badalona Barcelona (ES)
(74) Representative: Minoja, Fabrizio

(57) **Abstract**

A novel polymorph form of Dexketoprofen trometamol is disclosed.

The polymorph of the invention is particularly suited for the preparation of solid oral pharmaceutical forms, particularly tablets obtained by direct compression.

## Description

The present invention concerns new polymorphs of dexketoprofen trometamol.

Dexketoprofen trometamol, disclosed in EP 668851 and US 5,554,789, is an analgesic and anti-inflammatory drug characterized by advantageous pharmacokinetics and solubility properties with respect to Dexketoprofen or other salts thereof.

### BACKGROUND OF THE INVENTION

The process for the preparation disclosed in EP 668851 does not allow to draw any conclusion as to the existence of possible polymorphisms of this compound.

It is known that many compounds of pharmaceutical interest exhibit polymorphisms and that some polymorph or crystal forms may be preferred in view of different chemical-physical properties which may affect the workability of the compound and its formulation into pharmaceutical dosage forms, its pharmacokinetics profile and/or its stability and shelf-life.

A number of different polymorphic forms have been disclosed for instance for well-known and widely used drugs, for instance gabapentin, sertraline, ranitidine, cimetidine, biphosphonates, formoterol tartrate, carvedilol, paroxetine, ritonavir, atorvastatin, just to cite some of the most known cases.

Polymorphism may play an important role particularly in the preparation of bulk active ingredient as well as in the preparation of pharmaceutical formulations, particularly of oral formulations, where factors such as flowability, particles size, hygroscopic properties, chemical and physical stability, crystal forms, rate of dissolution. Each of the above properties may involve important technical consequences which have to be taken into account by pharmacologists, production and formulation specialists, quality controls and regulatory experts.

The different polymorphic forms may be distinguished by means of known analytical methods such as thermogravimetric analysis (TGA), differential scanning calorimetry (DSC), powder X-ray crystallography, solid state ¹³C NMR spectrometry and infrared and near infrared spectrometry.

### SUMMARY OF THE INVENTION

The present invention provides a novel polymorph form, hereinafter defined as form A, of dexketoprofen trometamol, pharmaceutical formulations comprising said polymorphic form as active ingredient as well as a process for its preparation.

Dexketoprofen trometamol form A is characterized by a X-Ray Powder Diffraction Pattern comprising peaks at about 5.14, 8.02, 9.83, 10.06, 10.37, 16.058, 16.294, 17.496, 19.584, 21.468, 26.861 degrees two-theta and may be distinguished from a different polymorph, hereinafter referred to as Dexketoprofen trometamol form B, which is obtained under different crystallization conditions and which was found to be unstable, converting over time into the more stable polymorph A.

The polymorph A may be obtained by a process characterized by crystallization under controlled rate of cooling.

The polymorph of the invention is particularly suited for the preparation of solid oral pharmaceutical forms, particularly tablets obtained by direct compression methods, in admixture with suitable excipients.

### BRIEF DESCRIPTION OF THE DRAWINGS

Figure 1 shows the PXRD pattern for Dexketoprofen trometamol form A.
Figure 2 shows the PXRD pattern for Dexketoprofen trometamol form B.
Figure 3 shows IR spectra of Dexketoprofen trometamol forms A and B.

### DETAILED DESCRIPTION OF THE INVENTION

Dexketoprofen trometamol form A can be obtained by crystallization induced by controlled rate of cooling of solutions of Dexketoprofen trometamol in ethanol:xylene mixtures. The term "controlled rate of cooling" is meant a rate of cooling lower than about 3.5°C/h -7°C/h, preferably from about 4°C/h to 6°C/h, to 18-22°C, followed by further cooling to 0-5°C for a period of at least 6-15 hours.

More particularly, the process for the preparation of Dexketoprofen trometamol form A of the invention comprises:
a) dissolving Dexketoprofen acid in ethanol;
b) adding tromethamine to the solution obtained in a);
c) heating the mixture to give a clear solution (T = 50 - 55°C);
d) adding xylene to the solution obtained in c), and heating until a complete solution is obtained;
e) cooling to 40-46°C and then further cooling to 18-22°C in 4-6 hours;
f) completing crystallization of polymorph A by further cooling to a temperature of 0-5°C which is maintained for further 6-15 hours;
g) recovering the crystallized polymorph A and drying under vacuum.

When a faster rate of cooling is applied, for instance more than 8-10°C per hour, another polymorphic form of Dextroprofen trometamol is obtained, named hereinafter form B.

Said form, characterized by X-Ray Powder Diffraction Pattern comprising peaks at about 4.44, 8.95, 10.67, 14.763, 17.112, 27.185 degrees two-theta, is less stable than form A according to the invention and converts gradually and spontaneously into the claimed form A over a period of several weeks or months. This is of course undesirable since any change of physical status may affect stability, quality control specifications and manufacturing processes of the final formulations for clinical use.

The process object of the present invention allows to obtain Dexketoprofen trometamol in substantially pure form.

The term "substantially pure" is used herein to refer to a compound wherein the percent by weight content is higher than 95%, preferably more than 97%, more preferably more than 99% and even more preferably even more than 99.9%, i.e. practically a polymorph A completely free from the polymorph B.

On the other hand, polymorph B, even though thermodynamically less stable than polymorph A and therefore converting into polymorph A itself, may be also useful for some specific applications, for instance where the solid state properties are not critical for the formulation of formulations to be administered in form of solutions, gels, ointments, syrups and the like, in view of its identical toxicological and pharmacodynamics properties.

The invention therefore also provides as a further object pharmaceutical compositions comprising a mixture of Dexketoprofen polymorphs A and B for use as active ingredient.

The solubility characteristics of the polymorphs are substantially similar.

In step a), Dexketoprofen acid is dissolved in ethanol, optionally containing from 1 to 10% of ketones such as 2-butanone. The concentration of this solution is not critical but is usually comprised between 0.4 to 0.8 kg/l, preferably from 0.5 to 0.7 kg/l. The dissolution requires heating for about 30 minutes at 45-55°C under stirring.

Tromethamine is then added in substantially equimolar amount in a small (1-5%) excess with respect to Dexketoprofen. The addition is preferably carried out after cooling the Dexketoprofen mixture to about 30-40°C. Further heating to 50-55°C is usually required in order to obtain a clear solution.

Xylene is then added to this solution, in volumetric ratios ranging from about 1:1 to 1:1.5, preferably from 1.1 to 1:1.4.

After a first cooling step to a temperature still well above the room temperature, typically in the range from 40 to 46°C, the solution is slowly cooled to 18-22°C at a rate of 3.5°C/h -7°C/h, preferably from about 4°C/h to 6°C/h. The initial cooling step from 50-55°C to 40-46°C may be likewise carried out at the same rate or even faster, if desired.

The slow rate of cooling defined above is continued until a temperature of 0-5°C is reached. This temperature is maintained for at least 6 hours, preferably for at least 10 hours, more preferably for at least 15 hours.

Recovery of the precipitate may then be effected by any conventional method such as centrifugation or filtration through paper, glass fiber or other membrane materials, or a clarifying agent such as celite.

The product may the be washed with xylene or other solvent wherein the salt is substantially insoluble and the product may be finally dried under vacuum for several hours at 40-50°C, until the desired content of residual solvent is achieved.

The conditions and time for transformation and other process parameters may of course be optimized by the skilled practitioners through routine experimentation.

The polymorph according to the invention may be advantageously formulated into pharmaceutical compositions by means of known and conventional methods and carriers.

Selection of specific excipients and their amounts may be readily determined according to experience and consideration of the common general knowledge and reference works in the field, e.g. "Remington: The Science and Practice of Pharmacy", Mack Publishing Co., NY, USA, 20th Ed.

Even though the polymorph of the invention may be used for compositions to be administered by any route, oral, parenteral, topical, transdermal or intra-mucosal, its use is particularly advantageous for the formulation of oral solid forms, particularly tablets obtained by the direct compression methods, avoiding therefore a wet granulation step which may affect the stability of the polymorphic form.

It is moreover known that direct compression produces a more uniform tablet without granules.

Excipients that are particularly suited for direct compression methods comprise maize starch, microcrystalline cellulose, spray dried lactose and colloidal silica.

The content of Dexketoprofen trometamol polymorph A in the formulations according to the invention may be comprised within wide limits and will usually be the same as that already approved by Health Authorities, in view of their safety and effectiveness. For example, a typical unit dosage for oral administration is 10-50 mg of Dexketoprofen trometamol polymorph A.

The following Examples will disclose the invention in more detail.

### EXAMPLE 1- Preparation of Dexketoprofen trometamol form A.

320 kg of Dexketoprofen acid were dissolved into 550 1 of ethanol containing 5% of 2-butanone. The complete dissolution was obtained by heating the suspension to 50-55°C and stirring at this temperature for about 30 minutes.

152 kg of tromethamine were then added to the solution cooled to 35-40°C, washing the tromethamine container with further 50 l of ethanol which were added to the mixture.

The reaction mixture was then heated again to 50-55°C until complete dissolution.

800 l of xylene were added to the clear solution and the temperature was raised again to 50-55°C until a clear solution was obtained. The precipitation mixture was then cooled to 44-46°C and was then cooled within 4-6 hours to 18-22°C. Cooling was continued at the same rate to 18-22°C and finally to 0-5°C. This temperature was kept for at least 15 hours, the precipitate was filtered, washed with 200 l of xylene and dried at 50°C under vacuum for 25 hours. 401 kg of Dexketoprofen trometamol polymorph A were obtained (yield 85%).

### EXAMPLE 2- Characterization of Dexketoprofen trometamol form A.

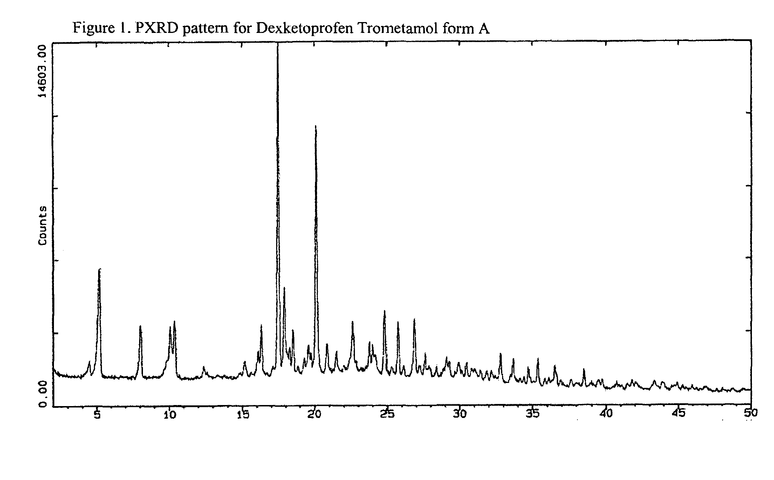

**a) The X-Ray Powder Diffraction** analysis was carried out on the product of Example 1 using the following instrument and experimental conditions:
*Debye-Scherrer INEL CPS-120* diffractometer.
120°C curved position sensitive detector. 4096 measuring channels.
(λ=1.5406 Å).
Working power: 40 kV-30mA.
Incident beam reflecting mirror and Ge (111) primary monochromator.
Beam height: 5mm; beam width: 0.3 mm.
Acquisition time: 3600 seconds.
Calibration by means of Na₂Ca₂AlF₁₃ as external standard and cubic spline function.
Linearization to a step size of 0.029° 2θ.
The spectrum is reported in the enclosed figure 1 whereas the most important characteristic peaks (2θ values in °, d spacings in A and relative intensities) are reported below:

| **2θ (°)** | **d (Å)** | **I/I₀** |
|---|---|---|
| 5.14 | 17.2 | 43 |
| 8.02 | 11.0 | 16 |
| 9.83 | 9.0 | 6 |
| 10.06 | 8.8 | 16 |
| 10.37 | 8.5 | 22 |
| 16.058 | 5.52 | 8 |
| 16.294 | 5.44 | 16 |
| 17.496 | 5.06 | 100 |
| 19.584 | 4.53 | 9 |
| 21.468 | 4.14 | 7 |
| 26.861 | 3.32 | 18 |

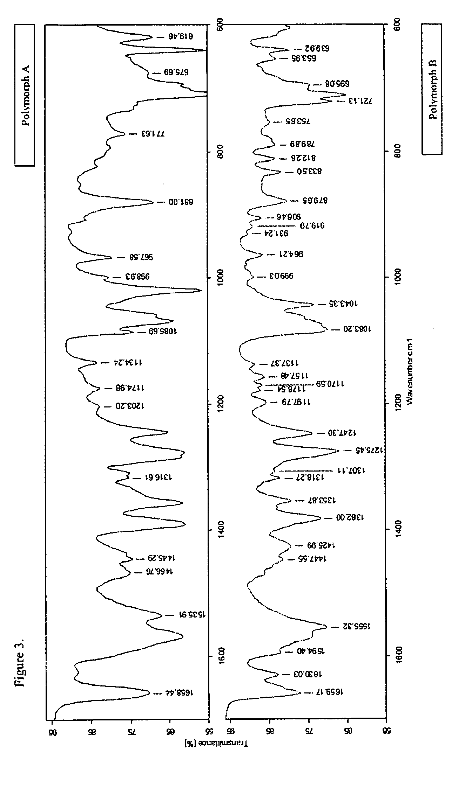

**b)** The **IR spectra** (enclosed in figure 3) have been recorded both by reflexion and in KBr tablet.
Intense band at 1020 cm⁻¹ is the most characteristic for the form A. Other differences between forms A and B are observed in the subintervals 1600-1500, 1100-1000 and 750-650 cm⁻¹.
**c) The Differential Scanning Calorimetry (DSC)** has been recorded between 80 and 120°C with an heating rate of 1°C /min. Dexketoprofen trometamol polymorph A shows fusion peak at 105.5-105.7°C, onset at 104.6-104.7°C and J/g values of 125-135.

### EXAMPLE 3- Preparation of Dexketoprofen trometamol form B.

7.5 kg of Dexketoprofen acid were dissolved into 13 l of ethanol containing 5% of 2-butanone. The complete dissolution was obtained by heating the suspension to 50-55°C and stirring at this temperature for about 30 minutes.

3.6 kg of tromethamine were then added to the solution cooled to 35-40°C, washing the trometamol container with 1 l of ethanol which was added to the mixture.

The reaction mixture was then heated again to 50-55°C until complete dissolution. 19.2 l of xylene were added to the clear solution and the temperature was raised again to 50-55°C until a clear solution was obtained. The precipitation mixture was then cooled to 40-45°C and then was quickly cooled to 0-5°C. This temperature was kept for no longer than 3 hours, the precipitate was filtered, washed with 3.5 l of xylene and dried at 45°C under vacuum for no longer than 25 hours. 9.3 kg of Dexketoprofen trometamol polymorph B were obtained (yield 84%).

### EXAMPLE 4- Characterization of Dexketoprofen trometamol form B.

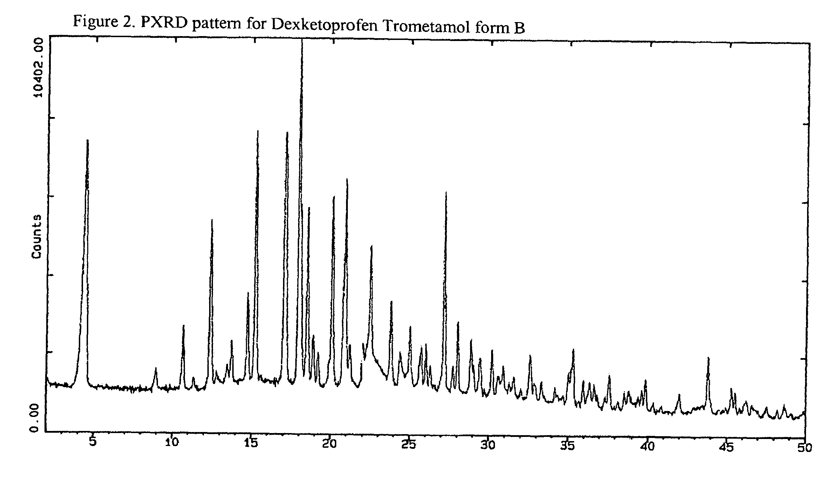

**a)** The **X-Ray Powder Diffraction** analysis was carried out on the product of Example 3 using the same conditions of Example 2. The spectrum is reported in the enclosed figure 2 whereas the most important characteristic peaks (2θ values in °, d spacings in Å and relative intensities) are reported below:

| **2θ (°)** | **d (Å)** | **I/I₀** |
|---|---|---|
| 4.44 | 19.9 | 73 |
| 8.95 | 9.9 | 6 |
| 10.67 | 8.3 | 19 |
| 14.763 | 6.00 | 27 |
| 17.112 | 5.18 | 74 |
| 27.185 | 3.28 | 58 |

**b)** The **IR spectra** (enclosed in figure 3) have been recorded both by reflexion and in KBr tablet.
A characteristic band at 1020 cm⁻¹ is not observed in the spectra of form B. Other differences between forms B and A are observed in the subintervals 1600-1500, 1100-1000 and 750-650 cm⁻¹.
**c) The Differential Scanning Calorimetry (DSC)** has been recorded between 80 and 120°C with an heating rate of 1°C /min. Dexketoprofen trometamol polymorph B shows fusion peak at 103.1- 103.3°C, onset at 102.1-102.5°C and J/g values of 110-120.

### EXAMPLE 5- Preparation of Dexketoprofen trometamol form A tablets by direct compression.

Dexketoprofen trometamol (71 g), Lactose (377 g.), maize starch (..13 g) and microcrystalline cellulose (19 g) were mixed for 20 minutes in a conventional blend mixer. Magnesium stearate (20 g) was then added to the mixture which was further mixed for 5 minutes.

The obtained blend was the subjected to direct compression by means of a rotatory tabletting machines with 9.75 mm punches.

Tablets having the following unitary compositions were obtained:

| | |
|---|---|
| Dexketoprofen trometamol | 36.9 mg |
| Maize starch | 6.7 mg |
| Microcrystalline cellulose | 10.0 mg |
| Lactose | 196.0 mg |
| Magnesium stearate | 10.4 mg |

## Claims

1. Dexketoprofen trometamol form A.

2. Dexketoprofen trometamol according to claim 1 **characterized by** a X-Ray Powder Diffraction Pattern comprising peaks at about 5.14, 8.02, 9.83, 10.06, 10.37, 16.058, 16.294, 17.496, 19.584, 21.468, 26.861 degrees two-theta.

3. A process for the preparation of Dexketoprofen trometamol form A of claims 1 or 2 comprising:
a) dissolving Dexketoprofen acid in ethanol;
b) adding tromethamine to the solution obtained in a);
c) heating the mixture to give a clear solution;
d) adding xylene to the solution obtained in c) and heating until a complete solution is obtained;
e) cooling to 40-46°C and then further cooling to 18-22°C in 4-6 hours;
f) completing crystallization of polymorph A by further cooling to a temperature of 0-5°C which is maintained for further 6-15 hours;
g) recovering the crystallized polymorph A and drying under vacuum.

4. Pharmaceutical compositions comprising as the active ingredient Dexketoprofen trometamol polymorph A and suitable carriers and/or excipients.

5. Pharmaceutical compositions according to claim 4 in form of tablets obtained by direct compression.

6. Pharmaceutical compositions according to claim 4 or 5 also comprising Dexketoprofen trometamol form B.
